# FASCICULE DE BREVET EUROPEEN

(11) **EP 2 493 545 B1**
(45) Date de publication et mention de la délivrance du brevet: **03.07.2013**
(21) Numéro de dépôt: 10770817.4
(22) Date de dépôt: 26.10.2010
(51) Int. Cl.: A61M 25/06, A61M 5/32

(54) **CAGE ANTI-PIQUE ET KIT DE PONCTION COMPRENANT UNE CAGE ANTI-PIQUE**
NADELSTICHSCHUTZ UND DURCHSTICHKIT MIT SOLCH EINEM NADELSTICHSCHUTZ
NEEDLE STICK GUARD, AND PUNCTURING KIT INCLUDING SUCH A NEEDLE STICK GUARD

(30) Priorité: 26.10.2009 FR 0957507
(43) Date de publication de la demande: 05.09.2012
(73) Titulaire: Vygon, 95440 Ecouen (FR)
(72) Inventeur: CARREZ, Jean-Luc, F-95440 Ecouen (FR); COUSSEGAL, Jean-Louis, F-95250 Beauchamp (FR); LETANG, Fabien, F-60127 Morienval (FR); GUYOMARC'H, Pierrick, F-95120 Ermont (FR)
(74) Mandataire: Regimbeau
(86) Numéro de dépôt international: PCT/EP2010/066119
(87) Numéro de publication internationale: WO 2011/051259

(56) Documents cités:
- EP-A2- 0 747 083
- WO-A1-01/78595
- WO-A1-2004/043521
- US-A1- 2008 065 015

## Description

### Domaine de l'invention

L'invention concerne une cage anti-pique pour kit de ponction anti-pique pour la pose d'un cathéter par la méthode de Seldinger.

L'invention concerne également un kit de ponction anti-pique pour la pose d'un cathéter par la méthode de Seldinger.

### État de la technique

La méthode de Seldinger est une méthode de pose d'un cathéter central en voie veineuse avec son extrémité distale au niveau de la veine aorte. Cette méthode permet également la cathétérisation d'une artère.

La pose d'un cathéter par la méthode de Seldinger comprend les étapes suivantes :
- l'introduction de l'aiguille dans une veine jusqu'à l'endroit souhaité ;
- l'introduction à l'intérieur de l'aiguille d'un guide jusqu'à l'endroit souhaité ;
- le retrait de l'aiguille hors de la veine ; le guide restant en place ;
- l'introduction d'un cathéter par coulissement le long du guide jusqu'à l'endroit souhaité ; et
- le retrait du guide.

Il est possible entre l'étape de retrait de l'aiguille et celle de l'introduction du cathéter d'agrandir l'incision effectuée par l'aiguille sur la peau en introduisant par coulissement le long du guide un dilatateur connu de l'homme du métier. Une fois l'incision agrandie, le dilatateur est retiré ; le guide restant en place.

La manipulation d'une aiguille, lors de la pose du cathéter par la méthode de Seldinger, présente un risque de blessure pour l'opérateur.

Les documents EP0747083 et US2008/065015, par exemple, décrivent des systèmes anti-pique comprenant un clapet présentent une ouverture dans laquelle est engagée l'aiguille. Lorsque l'aiguille est retirée, elle se désengage du clapet, entrainant le basculement de celui-ci qui vient former une barrière protégeant la pointe de l'aiguille. Ces systèmes ne peuvent pas être adaptés à la pose d'un cathéter par la méthode de Seldinger puisque le guide ne permettrait pas le basculement du clapet.

Le document WO2004/043521, quant à lui, décrit un système de protection comprenant une bascule présentant un orifice dans lequel est engagée l'aiguille de manière à ce que le retrait de l'aiguille entraine la rotation de la bascule et la libération du port du cathéter. Encore une fois, ce système ne peut pas être adapté a la pose d'un cathéter par la méthode de Seldinger, puisque le guide ne permettrait pas la rotation de la bascule.

Enfin, le document WO 01/78595 décrit un système de protection d'une séringue avec guide. L'aiguille est entourée d'une gaine de protection pouvant coulisser sur l'aiguille entre une position de protection, dans laquelle la gaine recouvre la pointe de l'aiguille, et une position de ponction dans laquelle la pointe de l'aiguille dépasse de la gaine. Lors de la pose, l'aiguille et la gaine sont insérées dans la veine ou l'artère du patient en position de ponction. La gaine est ensuite rétractée de manière à ce que la gaine recouvre la pointe en position de protection. L'aiguille peut alors être retirée du patient sans risque de pique accidentelle puisque la pointe de l'aiguille est recouverte par la gaine de protection. Le système décrit dans le document WO 01/78595 nécessite l'intervention de l'utilisateur pour réaliser l'étape de réalisation de la gaine.

### Présentation de l'invention

Un but de l'invention est de palier au moins un inconvénient de l'état de la technique.

Pour ce faire, l'invention propose une cage anti-pique pour kit de ponction anti-pique pour la pose d'un cathéter par la méthode de Seldinger selon la revendication 1.

D'autres caractéristiques optionnelles et non limitatives sont :
- la cage comprend en outre un élément résilient pour forcer la bascule vers l'intérieur du corps de la cage :
- l'orifice proximal présente un diamètre ajusté au diamètre du tube de l'aiguille ;
- l'orifice proximal est effectué sur une lame métallique, la lame métallique et l'élément résilient formant une pièce unique ;
- la cage comprend en outre un renfoncement sortant par rapport au corps et périphérique à l'orifice proximal pour la réception d'une déformation locale du tube de l'aiguille ; et
- la cage comprend en outre un orifice intermédiaire entre l'orifice proximal et l'orifice distal pour la stabilisation de l'aiguille par rapport à la cage anti-pique.

L'invention propose également un kit de ponction anti-pique pour la pose d'un cathéter par la méthode de Seldinger à un point de cathétérisation sur la peau d'un être vivant comprenant :
- une cage anti-pique selon l'un des modes de réalisation de l'invention ;
- un élément d'immobilisation pour immobiliser temporairement la cage anti-pique à proximité du point de cathétérisation ;
l'élément d'immobilisation comprenant une embase pour la réception d'une partie distale de la cage anti-pique et un ergot disposé sur l'embase et adapté pour être mis en prise par le bec externe de la cage anti-pique.

D'autres caractéristiques optionnelles et non limitatives sont :
- l'élément d'immobilisation comprend en outre un élément longitudinal relié à l'embase par une charnière pour le déplacement de l'élément longitudinal entre une position proximale et une position distale ;
   l'élément longitudinal étant au contact de la peau dans sa position distale et permettant l'immobilisation temporaire de la cage anti-pique par sa fixation sur la peau ;
- le kit de ponction comprend en outre un connecteur comportant :
   ■ un logement de cage pour la réception d'une partie proximale de la cage anti-pique ; et
   ■ un logement d'aiguille pour la réception d'une embase de l'aiguille ;
      le logement d'aiguille comprenant des moyens de connexion avec l'embase de l'aiguille ;
- le logement de cage du connecteur est un évidement ajusté à la cage anti-pique de manière à ce que la bascule soit bloquée dans la position de verrouillage quand la cage anti-pique est logée dans le logement de cage.

Un avantage de la cage anti-pique et du kit de ponction anti-pique selon l'invention est de permettre une manipulation du kit de ponction sans risque de piqûre pour l'opérateur sans ajouter des étapes de manipulation supplémentaires et contraignantes.

### Présentation des dessins

D'autres caractéristiques, buts et avantages apparaîtront à la lecture de la description détaillée ci-après, en référence aux dessins donnés à titre illustratif et non limitatif, parmi lesquels :
- la figure 1 est une vue en coupe longitudinale d'une cage anti-pique selon l'invention, et montrant la cage dans une première configuration ;
- la figure 2 est une vue en coupe longitudinale de la cage anti-pique de la figure 1, et montrant la cage dans une deuxième configuration ;
- la figure 3 est une vue en coupe longitudinale de la cage anti-pique de la figure 1, et montrant la cage dans une troisième configuration ;
- la figure 4 est une vue de trois-quarts de la cage anti-pique de la figure 1 ;
- la figure 5 est une vue de trois-quarts d'un élément d'immobilisation utilisé avec la cage anti-pique de la figure 1 ;
- la figure 6 est une vue en coupe longitudinale d'un kit de ponction anti-pique selon l'invention avec un élément d'immobilisation dans sa position proximale ;
- la figure 7 est une vue de profil du kit de ponction anti-pique de la figure 6 avec l'élément d'immobilisation dans sa position distale, au contact de la peau d'un être vivant.

### Description détaillée de l'invention

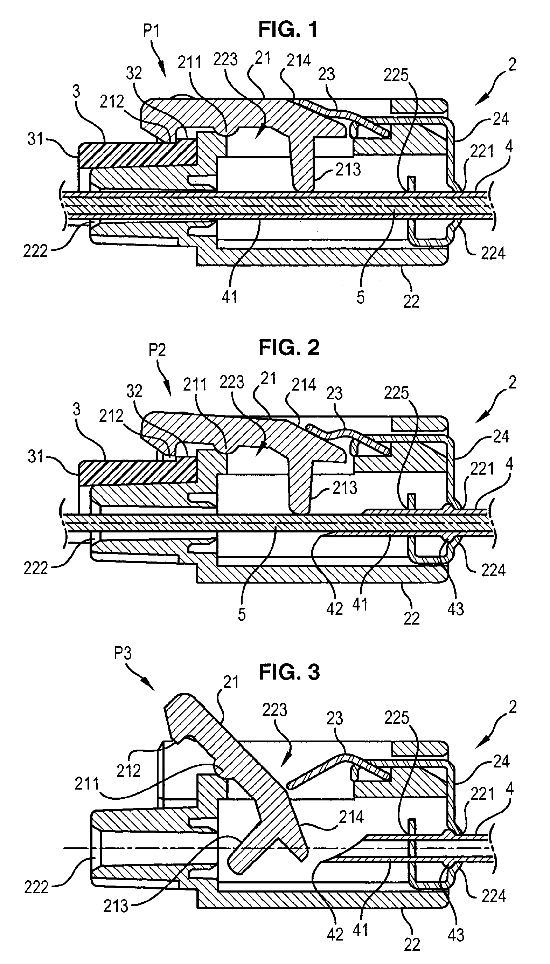

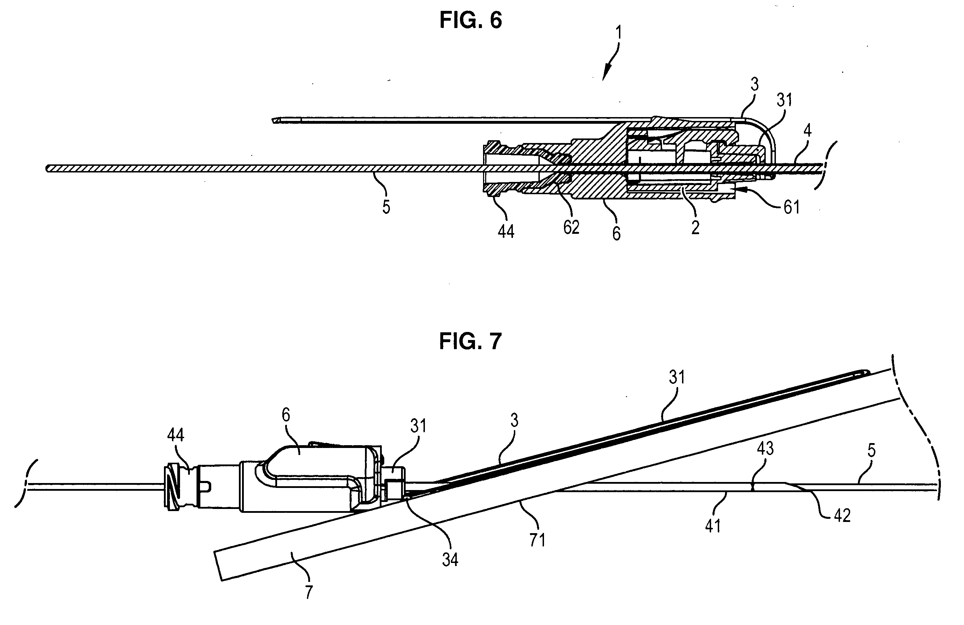

Un kit de ponction anti-pique et une cage anti-pique pour le kit de ponction anti-pique pour la pose d'un cathéter par la méthode de Seldinger sont ci-après décrits en référence aux figures 1 à 7.

Le kit de ponction anti-pique **1** selon l'invention comprend une cage anti-pique **2**.

Dans la suite de la description détaillée, les adjectifs « proximal » et « distal » désignent des positions relatives l'une par rapport à l'autre. La position proximale est celle qui est la plus proche du corps de l'opérateur tenant la cage anti-pique **2**. La position distale est celle qui est la plus éloignée du corps de l'opérateur tenant la cage anti-pique **2**.

La cage anti-pique **2** comprend un corps **22** comportant un orifice proximal **221** et un orifice distal **222** pour l'insertion d'un tube **41** d'une aiguille **4** et l'insertion d'un guide spiralé **5** entre les orifices proximal **221** et distal **222.**

Le corps **22** comporte également un évidement **223** pour la réception d'une bascule **21**, et un logement **224** en périphérie de l'évidement **223** pour la réception d'un tourillon **211** de la bascule **21**.

La cage anti-pique **2** comprend donc une bascule **21** présentant un tourillon **211** pour sa rotation autour d'un axe **21A**, et un bec externe **212** pour le verrouillage de la cage anti-pique 2 avec une embase **31** d'un autre élément du kit de ponction **1**.

L'autre élément peut être par exemple un élément d'immobilisation **3** de la cage anti-pique **2** et décrit plus en détail par la suite.

L'autre élément peut être tout autre élément dont le verrouillage avec la cage anti-pique **2** est nécessaire.

La bascule **21** présente trois positions distinctes **P1**, **P2**, **P3** autour de son axe de rotation **21A**.

Une position de verrouillage **P1** est représentée sur la figure 1. Dans cette position de verrouillage **P1**, le bec externe **212** verrouille la cage anti-pique **2** avec l'embase **31** de l'autre élément **3** du kit de ponction **1**.

Une position de déverrouillage P2 est représentée sur la figure 2. Dans cette position de déverrouillage **P2**, le bec externe **212** libère la cage anti-pique **2** d'avec l'autre élément **3** du kit de ponction **1**.

Une position de protection **P3** est représentée sur la figure 3. Dans cette position de protection **P3**, l'extrémité tranchante **42** de l'aiguille **4** est emprisonnée par le corps **22** et la bascule **21**.

Afin de commander la position de la bascule **21** autour de son axe de rotation **21 A**, la bascule **21** présente un talon **213** interne au corps **22**. Le talon **213** s'étend donc à l'intérieur de l'évidement **223**.

La commande de la position de la bascule **21** autour de son axe de rotation **21** A se fait par rapport à un diamètre externe du tube **41** de l'aiguille **4** et/ou du guide spiralé **5**. De manière plus détaillée, le talon **213**, le diamètre externe du tube **41** de l'aiguille **4** et le diamètre du guide spiralé **5** coopèrent de manière à ce que :
- la position de verrouillage **P1** soit obtenue quand le talon **213** repose sur le diamètre externe du tube **41** de l'aiguille **4** ;
- la position de déverrouillage **P2** soit obtenue quand le talon **213** repose sur le diamètre externe du guide spiralé **5** ; et
- la position de protection **P1** soit obtenue quand le talon **213** ne repose ni sur le diamètre externe du tube **41** de l'aiguille **4**, ni sur celui du guide spiralé **5**.

La disposition d'une bascule **21** présentant ces trois positions est intéressante pour un kit de ponction **1** anti-pique comprenant en outre, par exemple, un élément d'immobilisation **3** pour immobiliser temporairement la cage anti-pique **2** à proximité d'un point **71** de cathétérisation réalisé sur la peau **7** d'un être vivant.

L'élément d'immobilisation **3** permet de maintenir immobile la cage anti-pique **2** temporairement. Ainsi, lors du retrait de l'aiguille **4** le long de la cage anti-pique **2**, ceci permet d'éviter que la cage anti-pique **2** ne soit entraînée par le mouvement de retrait de l'aiguille **4**. Ceci est important pour amener l'extrémité tranchante **42** de l'aiguille **4** dans la cage anti-pique **2**.

L'élément d'immobilisation **3** peut comprendre une embase **31**, pour la réception d'une partie distale de la cage anti-pique **2**, et un ergot **32** disposé sur l'embase **31**. Cet ergot **32** est adapté pour être mis en prise par le bec externe **212** de la cage anti-pique **2** ; la mise en prise de l'ergot **32** avec le bec externe **212** permettant de verrouiller la cage anti-pique **2** avec l'élément d'immobilisation **3**.

Lors de la pose d'un cathéter par la méthode de Seldinger, le kit de ponction **1** est utilisé comme suit.

Tout d'abord, l'aiguille **4**, dont le tube est déjà inséré entre l'orifice proximal **221** et l'orifice distal **222** de la cage anti-pique **2**, est introduite à travers la peau **7** au point **71** de cathétérisation grâce à son extrémité tranchante **42**. Lors de l'introduction de l'aiguille **4**, le kit de ponction **1** est approché de la peau **7** jusqu'au point **71** de cathétérisation.

Le bec externe **212** est en prise avec l'ergot **32**. Par exemple, le bec externe **212** constitue une butée pour l'ergot **32** : l'élément d'immobilisation **3** ne peut être séparé de la cage anti-pique **2** comme illustré sur la figure 1 (représentant également le guide spiralé **5** déjà introduit, voir ci-dessous).

Une fois l'aiguille **4** en place, le guide spiralé **5** est inséré à l'intérieur de l'aiguille **4** (et donc à travers la cage anti-pique **2**) jusqu'à l'endroit souhaité dans une veine ou une artère. L'aiguille **4** doit ensuite être retirée, alors que la cage anti-pique **2** doit rester en place : ceci est rendu possible grâce à l'élément d'immobilisation **3**.

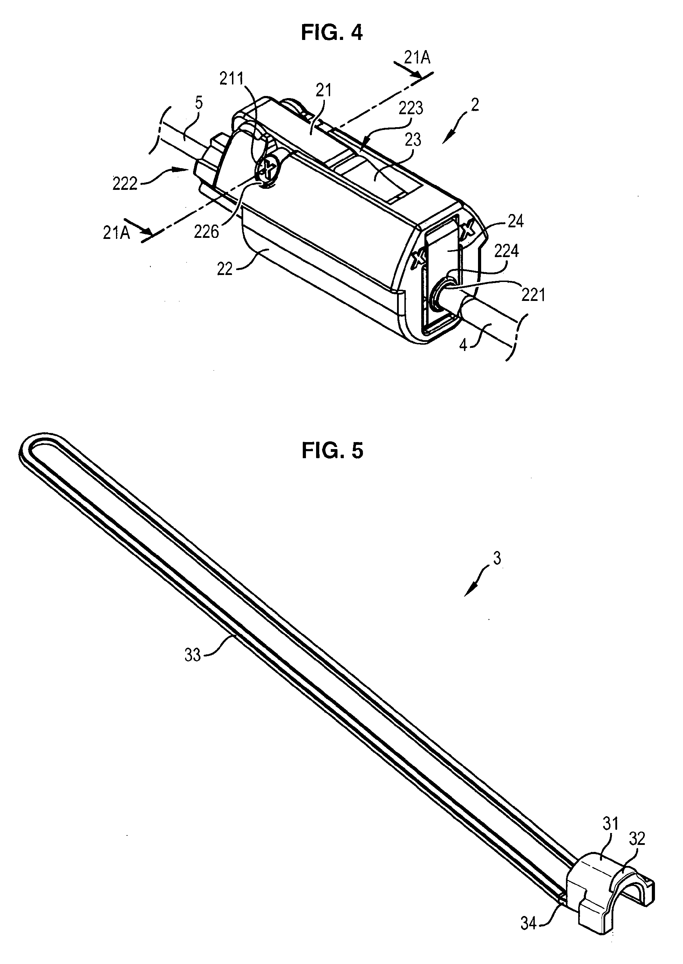

Par exemple, comme illustré sur la figure 5, l'élément d'immobilisation **3** comprend en outre un élément longitudinal **33** relié à l'embase **31** par une charnière **34** pour le déplacement de l'élément longitudinal **33** entre une position proximale et une position distale.

Lorsque le guide spiralé **5** est introduit dans l'aiguille **4** (dans le sens orifice proximal **221** vers orifice distal **222**) et mis en place, l'élément longitudinal **33**, au départ rabattu du côté de la cage anti-pique **4** (dans sa position proximale), est amené au contact de la peau **7** dans sa position distale. Sa fixation sur la peau **7** permet l'immobilisation temporaire de la cage anti-pique **2** puisque celle-ci est verrouillée avec l'élément d'immobilisation **3**.

La fixation de l'élément longitudinal **33** peut être assurée soit par simple pression de l'élément longitudinal **33** sur la peau **7** (pression effectuée par la main de l'opérateur), soit par l'utilisation d'un adhésif (sparadrap ou autre), soit par tout autre moyen adapté.

Lors du retrait de l'aiguille **4** (dans le sens orifice distal **222** vers orifice proximal **221**), le talon **213** de la bascule repose sur le diamètre externe du tube **41** de l'aiguille **4**. La cage anti-pique **2** est donc verrouillée avec l'élément d'immobilisation **3**.

Lorsque l'aiguille est suffisamment retirée, le talon **213** ne repose plus sur le diamètre externe du tube **41** de l'aiguille **4** mais sur le diamètre externe du guide spiralé **5** (position de déverrouillage **P2**, voir figure 2) : la bascule a alors subi une rotation autour de son axe de rotation **21A** du fait de son poids.

Cette rotation de la bascule **21** peut également être assurée grâce à un élément résilient **23** prévu sur la cage anti-pique **2** pour forcer la bascule **21** vers l'intérieur du corps **22** de la cage **2**.

Par exemple, la bascule **21** possède une surface **214** d'appui et l'élément résilient **23** qui peut être une lame en métal vient appuyer sur la surface **214** d'appui de manière à exercer une force vers l'intérieur de l'évidement **223**.

La force exercée est suffisamment faible pour que l'aiguille **4** puisse aisément être déplacée par coulissement entre l'orifice distal **222** et l'orifice proximal **221**, et plus tard pour que lors du retrait de la cage anti-pique **2** alors que le talon **213** repose sur le guide spiralé **5**, le guide spiralé **5** ne soit pas entraîné. Mais la force exercée doit être supérieure au poids de la bascule **21** et supérieure aux forces de frottement entre le tourillon **211** et le logement **224** de celui-ci.

La rotation de la bascule **21** entraîne un soulèvement du bec externe **212** libérant l'ergot **32** : l'ergot **32** n'étant plus en butée contre le bec externe **212**, la cage anti-pique **2** peut être éloignée de l'élément d'immobilisation **3**.

La cage anti-pique **2** est éloignée de l'élément d'immobilisation **3** par l'opérateur en continuant de tirer sur l'aiguille **4** de manière connu par l'homme du métier.

Par exemple, l'orifice proximal **221** de la cage anti-pique **2** présente un diamètre ajusté au diamètre du tube **41** de l'aiguille **4** et le tube **41** de l'aiguille **4** comprend une déformation locale **43** de manière à ce que le tube **41** présente suivant au moins une direction des dimensions supérieurs au diamètre de l'orifice proximal **221**. Par exemple, le tube **41** est écrasé à un endroit.

La distance entre l'extrémité tranchante **42** et la déformation locale **43** est choisie de manière à ce que l'extrémité tranchante **42** reste à l'intérieur de la cage anti-pique **2** lorsque le talon **213** de la bascule **21** n'est plus en contact avec le diamètre externe du tube **41** de l'aiguille **4**, et de manière à rendre possible la rotation de la bascule **21** autour de son axe de rotation **21 A** vers sa position de protection **P3**.

L'opérateur continue alors de retirer l'aiguille **4** entraînant la cage anti-pique **2** loin de l'élément d'immobilisation **3** jusqu'à ce que le guide spiralé 5 soit entièrement dégagé de la cage anti-pique **2** : le talon **213** ne reposant plus sur le diamètre externe du guide spiralé **5**.

Lorsque le talon **213** ne repose plus sur le diamètre externe du guide spiralé **5**, la bascule **21** subit une rotation autour de son axe de rotation **21A** vers l'intérieur de l'évidement **223** grâce à son poids et/ou éventuellement grâce à l'élément résilient **23** vers sa position de protection **P3** comme illustré sur la figure 3.

En position de protection **P3** la bascule empêche que l'extrémité tranchante **42** ne puisse ressortir de la cage anti-pique **2** suite à un déplacement de l'aiguille **4** dans le sens orifice proximal **221** vers orifice distal **222**. Les déplacements de l'aiguille **4** peuvent donc être contraints grâce à la bascule **21** d'une part et à la déformation locale **43** du tube **41** de l'aiguille **4**.

Lorsque la cage anti-pique **2** comprend l'élément résilient **23**, le fait que celui-ci soit choisi de manière à ce que la force qu'il exerce sur la surface **214** d'appui de la bascule **21** soit supérieure au poids de la bascule **21** permet d'empêcher que la bascule **21** ne sorte de l'évidement **223** si la cage anti-pique **2** vient à être retournée.

De manière optionnelle, l'orifice proximal **221** peut être effectué sur une lame métallique **24**, la lame métallique **24** et l'élément résilient **23** formant une pièce unique.

Ainsi quand l'opérateur retire l'aiguille **4** dans le sens orifice distal **222** vers orifice proximal **221**, la déformation locale **43** du tube **41** de l'aiguille **4** vient en butée sur la périphérie de l'orifice proximal **221** et déformer la lame métallique **24**. La déformation de la lame métallique **24** entraine l'application d'une force supplémentaire par l'élément résilient **23** sur la surface **214** d'appui de la bascule **21** favorisant d'autant plus la rotation de la bascule **21** autour de son axe de rotation **21A**.

La cage anti-pique **2** peut également comprendre, dans les cas où l'orifice proximal **221** est ajusté au diamètre externe du tube **41** de l'aiguille **4**, un renfoncement **224** sortant par rapport au corps **22** et périphérique à l'orifice proximal **221** pour la réception de la déformation locale **43** du tube **41** de l'aiguille **4**.

La cage anti-pique **2** peut encore comprendre un orifice intermédiaire **225** entre l'orifice proximal **221** et l'orifice distal **222** pour la stabilisation de l'aiguille **4** par rapport à la cage anti-pique **2**. La position de l'orifice intermédiaire **225** est choisie de manière à ce qu'il soit positionné entre l'orifice proximal **221** et la bascule **21** lorsque celle-ci est dans sa position de protection **P3**, le tube **41** de l'aiguille **4** traversant alors l'orifice proximal **221** et l'orifice intermédiaire **225**.

Le kit de ponction **1** anti-pique peut également comprendre un connecteur **6** comportant :
- un logement de cage **61** pour la réception d'une partie proximale de la cage anti-pique **2** ; et
- un logement d'aiguille **62** pour la réception d'une embase **44** de l'aiguille **4**.

Ainsi, grâce à ce connecteur **6**, le montage de l'aiguille **4** dans la cage anti-pique **2** est plus aisé. Aussi la manipulation de l'aiguille est facilitée et ce car l'opérateur peut manipuler le connecteur **6** dont les dimensions sont choisies pour être plus grandes que celles de l'embase **44** de l'aiguille **4.** En effet, lors du retrait de l'aiguille **4**, l'opérateur manipule le connecteur **6**.

Le logement d'aiguille **62** comprend des moyens de connexion avec l'embase **44** de l'aiguille **4**, par exemple un évidement du type Luer.

Le logement de cage **61** du connecteur **6** peut être un évidement ajusté à la cage anti-pique **2** de manière à ce que la bascule **21** soit bloquée dans la position de verrouillage **P1** quand la cage anti-pique **2** est logée dans le logement de cage **61**.

L'embase **44** de l'aiguille **4** peut présenter un cône (par exemple du type Luer) facilitant l'introduction du guide spiralé **5**.

## Revendications

1. Cage anti-pique (2) pour kit de ponction (1) anti-pique pour la pose d'un cathéter par la méthode de Seldinger à l'aide d'une aiguille et d'un guide spiralé, comprenant :
- une bascule (21) présentant un tourillon (211) pour sa rotation autour d'un axe (21A), et un bec externe (212) pour le verrouillage de la cage anti-pique (2) avec une embase (31) d'un autre élément (3) du kit de ponction (1) ;
- un corps (22) comportant :
- un orifice proximal (221) et un orifice distal (222) pour l'insertion d'un tube (41) d'une aiguille (4) et l'insertion d'un guide spiralé (5) entre les orifices proximal (221) et distal (222) ;
- un évidement (223) pour la réception de la bascule (21) ; et
- un logement (224) en périphérie de l'évidement (223) pour la réception du tourillon (211) de la bascule (21) ;
**caractérisée en ce que** la bascule (21) présente trois positions (P1, P2, P3) autour de son axe de rotation (21A) :
- une position de verrouillage (P1) dans laquelle le bec externe (212) verrouille la cage anti-pique (2) avec l'embase (31) de l'autre élément (3) du kit de ponction (1) ;
- une position de déverrouillage (P2) dans laquelle le bec externe (212) libère la cage anti-pique (2) d'avec l'autre élément (3) du kit de ponction (1) ; et
- une position de protection (P3) dans laquelle l'extrémité tranchante (42) de l'aiguille (4) est emprisonnée par le corps (22) et la bascule (21);
et **en ce que** la bascule (21) présente un talon (213) interne au corps (22) pour la commande en position de la bascule (21) par rapport à un diamètre externe du tube (41) de l'aiguille (4) et/ou du guide spiralé (5) ;
- la position de verrouillage (P1) étant obtenue quand le talon (213) repose sur le tube (41) de l'aiguille (4) ;
- la position de déverrouillage (P2) étant obtenue quand le talon (213) repose sur le guide spiralé (5) ; et
- la position de protection (P3) étant obtenue quand le talon (213) ne repose ni sur le tube (41) de l'aiguille (4), ni sur le guide spiralé (5).

2. Cage anti-pique (2) selon la revendication 1, comprenant en outre un élément résilient (23) pour forcer la bascule (21) vers l'intérieur du corps (22) de la cage (2).

3. Cage anti-pique (2) selon la revendication 1 ou 2, dans lequel l'orifice proximal (221) présente un diamètre ajusté au diamètre du tube (41) de l'aiguille (4).

4. Cage anti-pique (2) selon les revendications 2 et 3, dans lequel l'orifice proximal (221) est effectué sur une lame métallique (24), la lame métallique (24) et l'élément résilient (23) formant une pièce unique.

5. Cage anti-pique (2) selon la revendication 3 ou 4, comprenant en outre un renfoncement (224) sortant par rapport au corps (22) et périphérique à l'orifice proximal (221) pour la réception d'une déformation locale (43) du tube (41) de l'aiguille (4).

6. Cage anti-pique (2) selon l'une des revendications 1 à 5, comprenant en outre un orifice intermédiaire (225) entre l'orifice proximal (221) et l'orifice distal (222) pour la stabilisation de l'aiguille (4) par rapport à la cage anti-pique (2).

7. Kit de ponction (1) anti-pique pour la pose d'un cathéter par la méthode de Seldinger à un point (71) de cathétérisation sur la peau (7) d'un être vivant, **caractérisé en ce qu'**il comprenant :
- une cage anti-pique (2) selon l'une des revendications 1 à 6 ;
- un élément d'immobilisation (3) pour immobiliser temporairement la cage anti-pique (2) à proximité du point (71) de cathétérisation ;
l'élément d'immobilisation (3) comprenant une embase (31) pour la réception d'une partie distale de la cage anti-pique (2) et un ergot (32) disposé sur l'embase (31) et adapté pour être mis en prise par le bec externe (212) de la cage anti-pique (2).

8. Kit de ponction (1) anti-pique selon la revendication 7, dans lequel l'élément d'immobilisation (3) comprend en outre un élément longitudinal (33) relié à l'embase (31) par une charnière (34) pour le déplacement de l'élément longitudinal (33) entre une position proximale et une position distale ;
l'élément longitudinal (33) étant au contact de la peau (7) dans sa position distale et permettant l'immobilisation temporaire de la cage anti-pique (2) par sa fixation sur la peau (7).

9. Kit de ponction (1) anti-pique selon la revendication 7 ou 8, comprenant en outre un connecteur (6) comportant :
- un logement de cage (61) pour la réception d'une partie proximale de la cage anti-pique (2) ; et
- un logement d'aiguille (62) pour la réception d'une embase (44) de l'aiguille (4) ;
le logement d'aiguille (62) comprenant des moyens de connexion avec l'embase (44) de l'aiguille (4).

10. Kit de ponction (1) anti-pique selon la revendication 9, dans lequel le logement de cage (61) du connecteur (6) est un évidement ajusté à la cage anti-pique (2) de manière à ce que la bascule (21) soit bloquée dans la position de verrouillage (P1) quand la cage anti-pique (2) est logée dans le logement de cage (61).

## Patentansprüche

1. Stechschutzkäfig (2) für ein Stechschutz-Punktionskit (1) zum Verlegen eines Katheters durch die Seldinger-Methode mit Hilfe einer Nadel und einer spiralförmigen Führung, umfassend:
- eine Wippe (21), die einen Drehzapfen (211) für ihre Drehung um eine Achse (21A) und einen äußeren Schnabel (212) für die Verriegelung des Stechschutzkäfigs (2) mit einer Basis (31) eines anderen Elements (3) des Punktionskits (1) aufweist;
- einen Körper (22) mit:
• einer proximalen Öffnung (221) und einer distalen Öffnung (222) zum Einführen einer Röhre (41) einer Nadel (4) und zum Einführen einer spiralförmigen Führung (5) zwischen der proximalen (221) und der distalen Öffnung (222);
• einer Ausnehmung (223) für die Aufnahme der Wippe (21); und
• einem Sitz (224) am Umfang der Ausnehmung (223) für die Aufnahme des Drehzapfen (211) der Wippe (21);
**dadurch gekennzeichnet, dass** die Wippe (21) drei Positionen (P1, P2, P3) um ihre Drehachse (21A) aufweist:
- eine Verriegelungsposition (P1), in welcher der äußere Schnabel (212) den Stechschutzkäfig (2) mit der Basis (31) des anderen Elements (3) des Punktionskits (1) verriegelt;
- eine Entriegelungsposition (P2), in welcher der äußere Schnabel (212) den Stechschutzkäfig (2) von der Basis (31) des anderen Elements (3) des Punktionskits (1) freigibt; und
- eine Schutzposition (P3), in der das Schneidende (42) der Nadel (4) durch den Körper (22) und die Wippe (21) gefangen gehalten wird;
und dass die Wippe einen Absatz (213) im Inneren des Körpers (22) zum Steuern der Position der Wippe (21) relativ zu einem Außendurchmesser der Röhre (41) der Nadel (4) und/oder der spiralförmigen Führung (5) aufweist;
- wobei die Verriegelungsposition (P1) erhalten wird, wenn der Absatz (213) auf der Röhre (41) der Nadel (4) aufliegt;
- wobei die Entriegelungsposition (P2) erhalten wird, wenn der Absatz (213) auf der spiralförmigen Führung (5) aufliegt; und
- wobei die Schutzposition (P3) erhalten wird, wenn der Absatz (213) weder auf der Röhre (41) der Nadel (4), noch auf der spiralförmigen Führung (5) aufliegt.

2. Stechschutzkäfig (2) nach Anspruch 1, der ferner ein elastisches Element (23) umfasst, um die Wippe (21) in Richtung des Inneren des Körpers (22) des Käfigs (2) zu drängen.

3. Stechschutzkäfig (2) nach Anspruch 1 oder 2, wobei die proximale Öffnung (221) einen Durchmesser aufweist, der an den Durchmesser der Röhre (41) der Nadel (4) angepasst ist.

4. Stechschutzkäfig (2) nach Anspruch 2 und 3, wobei die proximale Öffnung (221) an einer metallischen Zunge (24) ausgebildet ist, wobei die metallische Zunge (24) und das elastische Element (23) ein einziges Stück bilden.

5. Stechschutzkäfig (2) nach Anspruch 3 oder 4, umfassend ferner eine Vertiefung (224), die relativ zu dem Körper (22) austritt und die proximale Öffnung (221) umgibt, für die Aufnahme einer lokalen Verformung (43) der Röhre (41) der Nadel (4).

6. Stechschutzkäfig (2) nach einem der Ansprüche 1 bis 5, umfassend ferner eine Öffnung (225), die zwischen der proximalen Öffnung (221) und der distalen Öffnung (222) liegt, für die Stabilisierung der Nadel (4) relativ zu dem Stechschutzkäfig (2).

7. Stechschutz-Punktionskit (1) zum Verlegen eines Katheters durch die Seldinger-Methode an einem Kathetisierungspunkt (71) auf der Haut (7) eines Lebewesens, **dadurch gekennzeichnet, dass** es Folgendes umfasst:
- einen Stechschutzkäfig (2) nach einem der Ansprüche 1 bis 6;
- ein Feststellelement (3), um den Stechschutzkäfig (2) in der Nähe des Kathetisierungspunkts (71) vorübergehend festzustellen;
wobei das Feststellelement (3) eine Basis (31) für die Aufnahme eines distalen Teils des Stechschutzkäfigs (2) und einen Vorsprung (32) umfasst, der an der Basis (31) angeordnet ist und dafür geeignet ist, durch den äußeren Schnabel (212) des Stechschutzkäfigs (2) in Eingriff genommen zu werden.

8. Stechschutz-Punktionskit (1) nach Anspruch 7, wobei das Feststellelement (3) ferner ein längliches Element (33) umfasst, das mit der Basis (31) über ein Scharnier (34) für die Bewegung des länglichen Elements (33) zwischen einer proximalen Position und einer distalen Position verbunden ist;
wobei das längliche Element (33) in seiner distalen Position in Kontakt mit der Haut (7) ist und die vorübergehende Feststellung des Stechschutzkäfigs (2) durch seine Fixierung auf der Haut (7) ermöglicht.

9. Stechschutz-Punktionskit (1) nach Anspruch 7 oder 8, umfassend ferner einen Verbinder (6) mit:
- einem Käfigsitz (61) für die Aufnahme eines proximalen Teils des Stechschutzkäfigs (2);
- einem Nadelsitz (62) für die Aufnahme einer Basis (44) der Nadel (4);
wobei der Nadelsitz (62) Mittel für die Verbindung mit der Basis (44) der Nadel (4) umfasst.

10. Stechschutz-Punktionskit (1) nach Anspruch 9, wobei der Käfigsitz (61) des Verbinders (6) eine Ausnehmung ist, die an den Stechschutzkäfig (2) so angepasst ist, dass die Wippe (21) in der Verriegelungsposition (P1) gesperrt ist, wenn der Stechschutzkäfig (2) in dem Käfigsitz (61) aufgenommen ist.

## Claims

1. A needle stick guard (2) for an anti-needle-stick puncturing kit (1) for installing a catheter by the Seldinger method, with a needle and a spiral guide, comprising:
- a rocker (21) having a bearing (211) for its rotation about an axis (21 A), and an external tip (212) for locking the needle stick guard (2) together with a base (31) of another element (3) of the puncturing kit (1);
- a body (22) comprising:
- a proximal hole (221) and a distal hole (222) for inserting a tube (41) of a needle (4) and inserting a spiral guide (5) between the proximal (221) and distal (222) holes;
- a slot (223) for receiving the rocker (21); and
- a housing (224) on the periphery of the slot (223) for receiving the bearing (211) of the rocker (21);
**characterized in that** the rocker (21) has three positions (P1, P2, P3) about its axis of rotation (21 A) :
- a locking position (P1) wherein the external tip (212) locks the needle stick guard (2) together with the base (31) of the other element (3) of the puncturing kit (1);
- an unlocking position (P2) wherein the external tip (212) releases the needle stick guard (2) from the other element (3) of the puncturing kit (1); and
- a protection position (P3),wherein the sharp end (42) of the needle (4) is trapped by the body (22) of the rocker (21);
and **in that** the rocker (21) has a spur (213) within the body (22) for controlling the position of the rocker (21) with respect to an outer diameter of the tube (41) of the needle (4) and/or of the spiral guide (5);
- the locking position (P1) being obtained when the spur (213) is resting on the tube (41) of the needle (4);
- the unlocking position (P2) being obtained when the spur (213) is resting on the spiral guide (5); and
- the protection position (P3) being obtained when the spur (213) is resting neither on the tube (41) of the needle (4) nor on the spiral guide (5).

2. A needle stick guard (2) according to Claim 1, further including a resilient element (23) for forcing the rocker (21) toward the inside of the body (22) of the guard (2).

3. A needle stick guard (2) according to Claim 1 or 2, wherein the proximal hole (221) has a diameter corresponding to the diameter of the tube (41) of the needle (4).

4. A needle stick guard (2) according to Claims 2 and 3, wherein the proximal hole (221) is made in a metal strip (24), the metal strip (24) and the resilient element (23) constituting a single element.

5. A needle stick guard (2) according to Claim 3 or 4, further including an indentation (224), which protrudes from the body (22) and surrounds the proximal hole (221), for receiving a local distortion (43) of the tube (41) of the needle (4).

6. A needle stick guard (2) according to one of Claims 1 through 5, further including an intermediate hole (225) between the proximal hole (221) and the distal hole (222) for stabilizing the needle (4) with respect to the needle stick guard (2).

7. An anti-needle-stick puncturing kit (1) for installing a catheter by the Seldinger method at a catheterization point (71) on the skin (7) of a living being, **characterized in that** it comprises:
- a needle stick guard (2) according to one of Claims 1 to 6;
- an immobilization element (3) for temporarily immobilizing the needle stick guard (2) close to the catheterization point (71);
the immobilization element (3) including a base (31) for receiving a distal portion of the needle stick guard (2) and a ridge (32) arranged on the base (31) and adapted to be seized by the external tip (212) of the needle stick guard (2).

8. An anti-needle-stick puncturing kit (1) according to Claim 7, wherein the immobilization element (3) further includes a longitudinal element (33) connected to the base (31) by a hinge (34) for moving the longitudinal element (33) between a proximal position and a distal position;
the longitudinal element (33) being in contact with the skin (7) in its distal position and allowing a temporary immobilization of the needle stick guard (2) by attaching it to the skin (7).

9. An anti-needle-stick puncturing kit (1) according to Claim 7 or 8, further including a connector (6) comprising:
- a guard housing (61) for receiving a distal portion of the needle stick guard (2); and
- a needle housing (62) for receiving a base (44) of the needle (4);
the needle housing (62) including means for connecting with the base (44) of the needle (4).

10. An anti-needle-stick puncturing kit (1) according to Claim 9, wherein the guard housing (61) of the connector (6) is a recess fitted to the needle stick guard (2) so as to block the rocker (21) in the locking position (P1) when the needle stick guard (2) is housed in the guard housing (61).
